# EUROPEAN PATENT APPLICATION

(11) **EP 2 889 383 A1**
(43) Date of publication of application: **01.07.2015**
(21) Application number: 13832195.5
(22) Date of filing: 27.08.2013
(51) Int. Cl.: C12Q 1/68, G01N 33/50, G01N 33/53, C12N 15/09

(54) **DIAGNOSIS OF RHEUMATOID ARTHRITIS BY MICRORNA**

(30) Priority: 27.08.2012 JP 2012186937
(71) Applicant: Kyoto University, Sakyo-ku Kyoto-shi Kyoto 606-8501 (JP)
(72) Inventor: YOSHITOMI, Hiroyuki, Kyoto-shi Kyoto 606-8501 (JP); MURATA, Koichi, Kyoto-shi Kyoto 606-8501 (JP); MATSUDA, Shuichi, Kyoto-shi Kyoto 606-8501 (JP); ITO, Hiromu, Kyoto-shi Kyoto 606-8501 (JP); FURU, Moritoshi, Kyoto-shi Kyoto 606-8501 (JP)
(74) Representative: Tuxworth, Pamela M.
(86) International application number: PCT/JP2013/072919
(87) International publication number: WO 2014/034685

(57) **Abstract**

The present invention provides a method of testing a sample derived from the blood of a test subject for determining the pathology of rheumatoid arthritis, including the steps of (a) measuring an expression level of at least one miRNA selected from the group consisting of miR-24, miR-26a, miR-28-5p, miR-28-3p, miR-30c, miR-30e-3p, miR-125a-5p, miR-126-3p and miR-502-5p, or expression levels of miR-24, miR-30a-5p and miR-125a-5p, in a sample derived from the blood of a test subject, and (b) correlating the expression level measured in step (a) with the pathology of rheumatoid arthritis.

## Description

### Technical Field

The present invention relates to a method of testing a sample derived from the blood of a test subject for the determination of the pathology of rheumatoid arthritis, a diagnostic reagent to be used therefor and the like.

### Background Art

Rheumatoid arthritis (RA) is a systemic inflammatory disease characterized by destructive arthritis. Its morbidity rate is said to be 1% of the whole world's population, and 0.7-1 million patients reside in Japan, with 15,000 people newly developing the disease every year. In recent years, it has been clarified that the irreversible articular destruction in rheumatoid arthritis starts earlier than conventionally predicted, and therefore, positive use of a biological preparation exhibiting a high articular destruction suppressive action from an early stage has been recommended, which increases the importance of early diagnosis of rheumatoid arthritis.

MicroRNA (miRNA) is a short chain RNA molecule with about 22-base length, and suppresses translation of the target gene by binding to a complementary sequence in the 3'-untranslated region of the messenger RNA thereof. miRNA is known to regulate various biological phenomena, and 1/3 of the entire genes is assumed to be under expression regulation by miRNA. In 2008, it was shown that miRNA (miR-141) is present in plasma in which RNA has long been considered absent due to the presence of RNA degrading enzymes, and is available as a biomarker for prostate cancer (non-patent document 1).

On the other hand, the present inventors have clarified that rheumatoid arthritis can be determined and the state of the disease can be judged by measuring the concentrations of miR-16, miR-132, miR-146a, miR-155 and miR-223, in plasma, which miRNAs have been reported to be present intracellularly and related to rheumatoid arthritis (patent document 1).

### [Document List]

### [patent document]

patent document 1: WO 2011/126105 [non-patent document]
non-patent document 1: Proc Natl Acad Sci USA 2008, 105: 10513-10518

### SUMMARY OF THE INVENTION

### Problems to be Solved by the Invention

According to the new classification standard for rheumatoid arthritis published in 2010, since the significant emphasis is on the scores of rheumatism factor and anti-CCP antibody in the scoring for diagnosis, a case positive for these inspection items can be diagnosed to have rheumatoid arthritis even when articular tumentia is less, whereas a negative case cannot be diagnosed to have rheumatoid arthritis unless articular tumentia is found in 11 parts or more. Of the rheumatoid arthritis patients in Japan allegedly mounting to 0.7-1 million people, 30 percent are predicted to be negative for anti-CCP antibody. Since, according to the current diagnosis standard, the diagnosis is delayed in the anti-CCP antibody-negative cases, a diagnosis marker enabling early diagnosis of rheumatoid arthritis even in anti-CCP antibody-negative cases is demanded.

In view of the above situation, the present invention aims to establish a rheumatoid arthritis marker belonging to a category different from that of autoantibodies such as anti-CCP antibody, rheumatism factor and the like, particularly a marker applicable to autoantibody-negative cases as well, and provide a means for determining the presence or absence of affection with rheumatoid arthritis and the progression of symptoms (i.e., state of the disease) in rheumatoid arthritis patients.

### Means of Solving the Problems

In an attempt to achieve the above-mentioned object, the present inventors comprehensively studied miRNA concentration in plasma of rheumatoid arthritis patients and healthy humans. As a result, they have found miRNAs (miR-24, miR-26a, miR-28-5p, miR-28-3p, miR-30c, miR-125a-5p, miR-126-3p and miR-502-5p) that show significantly higher expression levels in the plasma of rheumatoid arthritis patients than those of healthy humans. When miR-24, miR-26a and miR-125a-5p from these miRNAs were used as markers, the sensitivity and specificity in the determination of rheumatoid arthritis were 63.7%/89.5%, 53.9%/94.3% and 64.7%/89.5%, respectively, with the optimal cutoff values. The sensitivity and specificity of rheumatism factor and anti-CCP antibody, which are the most famous markers conventionally used for the determination of rheumatoid arthritis, have been reported to be 54%/91% and 48%/98%, respectively (ARTHRITIS & RHEUMATISM 2000, 43(1): 155-163). Since none of miR-24, miR-26a and miR-125a-5p has heretofore been reported to be related to a disease, the high sensitivity and specificity comparable to those of well-known rheumatoid arthritis determination markers were completely unpredictable. In addition, using a composite value of three miRNAs of miR-24, miR-30a-5p and miR-125a-5p in combination (ePRAM, estimated Probability of RA using MicroRNA), a test with sensitivity of 78.4%, specificity of 92.3% could be performed, and further, a test with sensitivity of 94.1%, specificity of 95.2% could be performed using an existing anti-CCP antibody and these three miRNAs in combination. Furthermore, no remarkable difference in the expression level of miR-24, miR-26a and miR-125a-5p was observed between an anti-CCP antibody positive case and a negative case, which has clarified that the miRNAs are also useful for the determination of rheumatoid arthritis in anti-CCP antibody-negative cases.

The present inventors also studied the correlation between the absolute value of the concentration of miRNA in the plasma of rheumatoid arthritis patients or logarithm thereof, and the indices (VAS, DAS28 (ESR) and DAS28 (CRP)) of rheumatoid arthritis. As a result, correlation was found between VAS and miR-24, miR-26a, miR-30c, miR-126-3p and miR-502-5p; DAS28 (ESR) and miR-24, miR-26a, miR-30c, miR-30e-3p, miR-126-3p and miR-502-5p; as well as DAS28 (CRP) and miR-24, miR-26a, miR-30c, miR-30e-3p, miR-126-3p and miR-502-5p, and these miRNAs were found to be available as disease state markers. The relationship between these miRNAs and rheumatoid arthritis has not been reported at all heretofore.

The present inventors have conducted further studies based on these findings and completed the present invention.

Accordingly, the present invention is as described below.
[1] A method of testing a sample derived from the blood of a test subject for determining the pathology of rheumatoid arthritis, comprising a step of
   (a) measuring an expression level of at least one miRNA selected from the group consisting of miR-24, miR-26a, miR-28-5p, miR-28-3p, miR-30c, miR-30e-3p, miR-125a-5p, miR-126-3p and miR-502-5p, or expression levels of miR-24 and miR-125a-5p, in the sample derived from the blood of the test subject, and normalizing the expression level(s) with an expression level of an internal standard miRNA in the sample.
[2] The method of [1], further comprising the following step:
   (b) correlating the expression level measured in step (a) with the pathology of rheumatoid arthritis.
[3] The method of [1] or [2], wherein, in step (a), an expression level of at least one miRNA selected from the group consisting of miR-24, miR-26a and miR-125a-5p, or expression levels of miR-24 and miR-125a-5p, in the sample derived from the blood of the test subject, is/are measured.
[4] The method of [3], wherein, in step (a), expression levels of miR-24 and miR-125a-5p in the sample derived from the blood of the test subject are measured.
[5] The method of [3] or [4], further comprising a step of (a') measuring a level of an anti-CCP antibody in the sample derived from the blood of the test subject, wherein, in step (b), the expression level measured in step (a) and the anti-CCP antibody level measured in step (a') are correlated with the pathology of rheumatoid arthritis.
[6] The method of [3] or [4], wherein the test subject is anti-CCP antibody negative.
[7] The method of any of [1] - [6], wherein the sample derived from the blood is plasma.
[8] The method of any of [1] - [7], wherein the test subject is a human.
[9] A diagnostic reagent for determining the pathology of rheumatoid arthritis, comprising a nucleic acid probe or nucleic acid primer capable of specifically detecting at least one miRNA selected from the group consisting of miR-24, miR-26a, miR-28-5p, miR-28-3p, miR-30c, miR-30e-3p, miR-125a-5p, miR-126-3p and miR-502-5p, or
   a nucleic acid probe or nucleic acid primer capable of specifically detecting miR-24,
   and
   a nucleic acid probe or nucleic acid primer capable of specifically detecting miR-125a-5p.
[10] The diagnostic reagent of [9], comprising the nucleic acid probe or nucleic acid primer capable of specifically detecting at least one miRNA selected from the group consisting of miR-24, miR-26a and miR-125a-5p, or
   the nucleic acid probe or nucleic acid primer capable of specifically detecting miR-24,
   and
   the nucleic acid probe or nucleic acid primer capable of specifically detecting miR-125a-5p.
[11] The diagnostic reagent of [10], comprising the nucleic acid probe or nucleic acid primer capable of specifically detecting miR-24,
   and
   the nucleic acid probe or nucleic acid primer capable of specifically detecting miR-125a-5p.
[12] The diagnostic reagent of any of [9] - [11], further comprising a cyclic citrullinated peptide.
[13] The method of any of [1] - [8], wherein the internal standard miRNA is miR-30a-5p.
[14] The diagnostic reagent of any of [9] - [12], further comprising a nucleic acid probe or nucleic acid primer capable of specifically detecting miR-30a-5p.

[1'] A method of testing a sample derived from the blood of a test subject for determining the pathology of rheumatoid arthritis, comprising steps of
   (a) measuring an expression level of at least one miRNA selected from the group consisting of miR-24, miR-26a, miR-28-5p, miR-28-3p, miR-30c, miR-30e-3p, miR-125a-5p, miR-126-3p and miR-502-5p, or expression levels of miR-24, miR-30a-5p and miR-125a-5p, in the sample derived from the blood of the test subject, and
   (b) correlating the expression level(s) measured in step (a) with the pathology of rheumatoid arthritis.
[2'] The method of [1'], wherein, in step (a), the expression level of at least one miRNA selected from the group consisting of miR-24, miR-26a and miR-125a-5p, or expression levels of miR-24, miR-30a-5p and miR-125a-5p, in the sample derived from the blood of the test subject, is/are measured.
[3'] The method of [2'], wherein, in step (a), expression levels of miR-24, miR-30a-5p and miR-125a-5p in the sample derived from the blood of the test subject are measured.
[4'] The method of [2'] or [3'], further comprising a step of (a') measuring a level of an anti-CCP antibody in the sample derived from the blood of the test subject, wherein, in step (b), the expression level measured in step (a) and the anti-CCP antibody level measured in step (a') are correlated with the pathology of rheumatoid arthritis.
[5'] The method of [2'] or [3'], wherein the test subject is anti-CCP antibody-negative.
[6'] The method of any of [1'] - [5'], wherein the sample derived from the blood is plasma.
[7'] The method of any of [1'] - [6'], wherein the test subject is a human.
[8'] A diagnostic reagent for determining the pathology of rheumatoid arthritis, comprising a nucleic acid probe or
   nucleic acid primer capable of specifically detecting at least one miRNA selected from the group consisting of miR-24, miR-26a, miR-28-5p, miR-28-3p, miR-30c, miR-30e-3p, miR-125a-5p, miR-126-3p and miR-502-5p, or
   a nucleic acid probe or nucleic acid primer capable of specifically detecting miR-24 ,
   a nucleic acid probe or nucleic acid primer capable of specifically detecting miR-30a-5p, and
   a nucleic acid probe or nucleic acid primer capable of specifically detecting miR-125a-5p.
[9'] The diagnostic reagent of [8'], comprising the nucleic acid probe or nucleic acid primer capable of specifically detecting at least one miRNA selected from the group consisting of miR-24, miR-26a and miR-125a-5p, or
   the nucleic acid probe or nucleic acid primer capable of specifically detecting miR-24,
   the nucleic acid probe or nucleic acid primer capable of specifically detecting miR-30a-5p, and
   the nucleic acid probe or nucleic acid primer capable of specifically detecting miR-125a-5p.
[10'] The diagnostic reagent of [9'], comprising the nucleic acid probe or nucleic acid primer capable of specifically detecting miR-24,
   the nucleic acid probe or nucleic acid primer capable of specifically detecting miR-30a-5p, and
   the nucleic acid probe or nucleic acid primer capable of specifically detecting miR-125a-5p.
[11'] The diagnostic reagent of any of [8'] - [10'], further comprising a cyclic citrullinated peptide.

### Effect of the Invention

The present invention makes it possible to determine the presence or absence of affection with rheumatoid arthritis and determine the disease state of rheumatoid arthritis patients, without utilizing conventional markers such as rheumatism factor and anti-CCP antibody. Since high sensitivity and specificity comparable to those of conventional markers can be obtained, the present invention can accurately determine the presence or absence of affection with rheumatoid arthritis. Moreover, the present invention can determine the presence or absence of affection with rheumatoid arthritis at an early stage even in patients for whom early diagnosis of rheumatoid arthritis is conventionally difficult since they are anti-CCP antibody negative.

### Brief Description of the Drawings

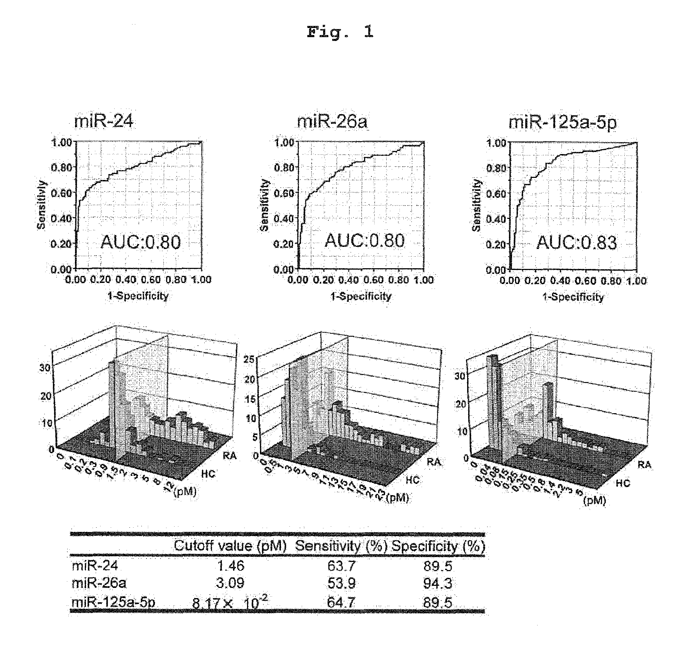

Fig. 1 shows the results of ROC curve analyses for miR-24, miR-26a and miR-125a-5p to distinguish rheumatoid arthritis patients (RA) from healthy individuals (HC). (upper panels) ROC curve. The vertical axis shows sensitivity and the horizontal axis shows 1-specificity. (lower panels) Concentration distribution of miRNA in plasma samples from RA and HC. The vertical axis shows sample number, and the horizontal axis shows concentration (pM) in plasma. (lower table) Cutoff value, sensitivity and specificity for each miRNA.

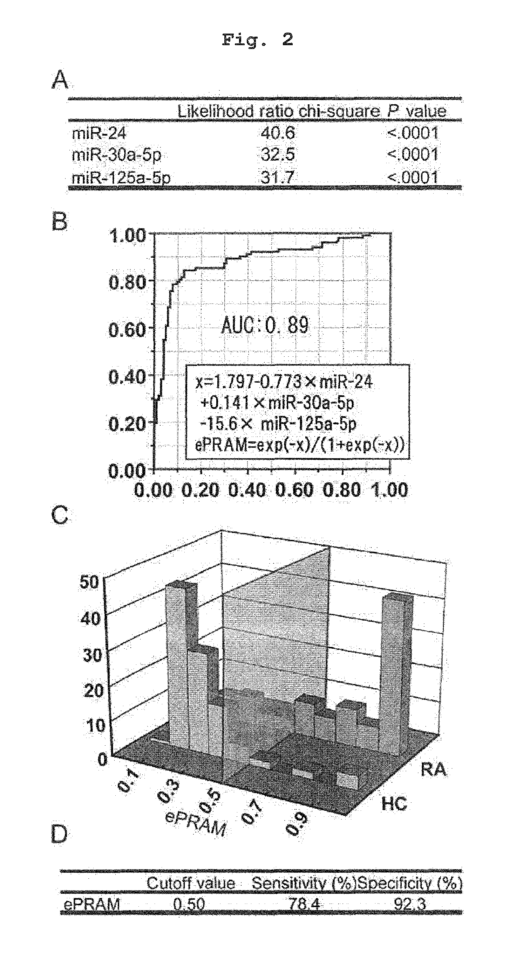

Fig. 2 shows the results of ROC curve analyses for composite values of 3 miRNAs of miR-24, miR-30a-5p and miR-125a-5p in combination. (A) Likelihood ratio chi-square of each miRNA and p-value. (B) ROC curve. The vertical axis shows sensitivity and the horizontal axis shows 1-specificity. (C) Distribution of composite values of plasma samples from RA and HC. The vertical axis shows sample number, and the horizontal axis shows composite value (ePRAM). (D) Cutoff value, sensitivity and specificity for ePRAM.

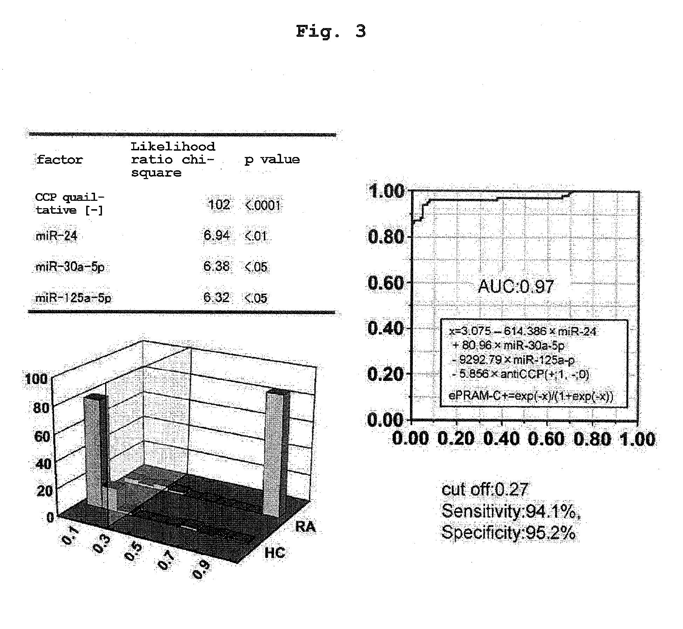

Fig. 3 shows the results of ROC curve analysis for composite values (ePRAM-C) of the presence or absence of an anti-CCP antibody, and 3 miRNAs of miR-24, miR-30a-5p and miR-125a-5p in combination. (upper left table) Likelihood ratio chi-square and p-value of CCP qualitative[-] and each miRNA. (lower left panel) Distribution of composite values of plasma samples from RA and HC. The vertical axis shows sample number, and the horizontal axis shows ePRAM-C. The dotted line shows cutoff value. (right panel) ROC curve. The vertical axis shows sensitivity, and the horizontal axis shows 1-specificity.

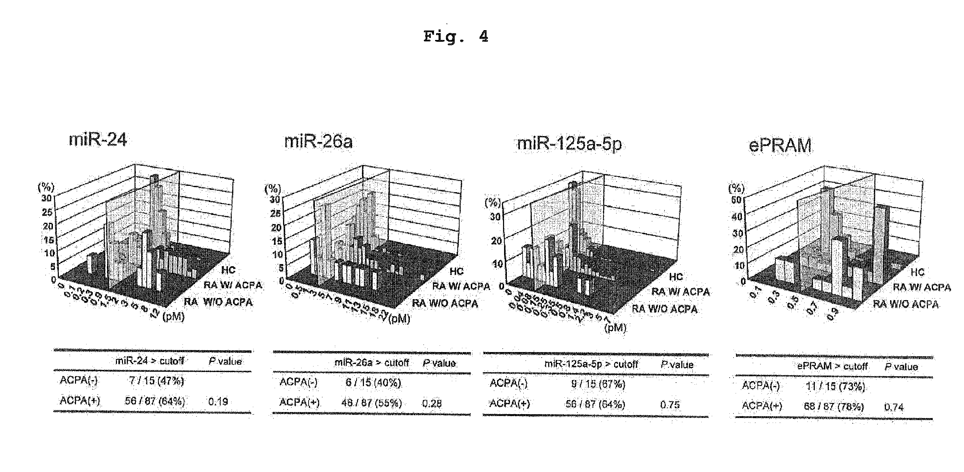

Fig. 4 shows distribution of concentrations of miR-24, miR-26a and miR-125a-5p in plasma samples from RA (anti-CCP antibody (ACPA)-positive case), RA (anti-CCP antibody-negative case) and HC, as well as distribution of composite values (ePRAM) of miR-24, miR-30a-5p and miR-125a-5p. The vertical axis shows the ratio (%) of the number of samples to the total number of samples of each group, and the horizontal axis shows concentration in plasma or composite value.

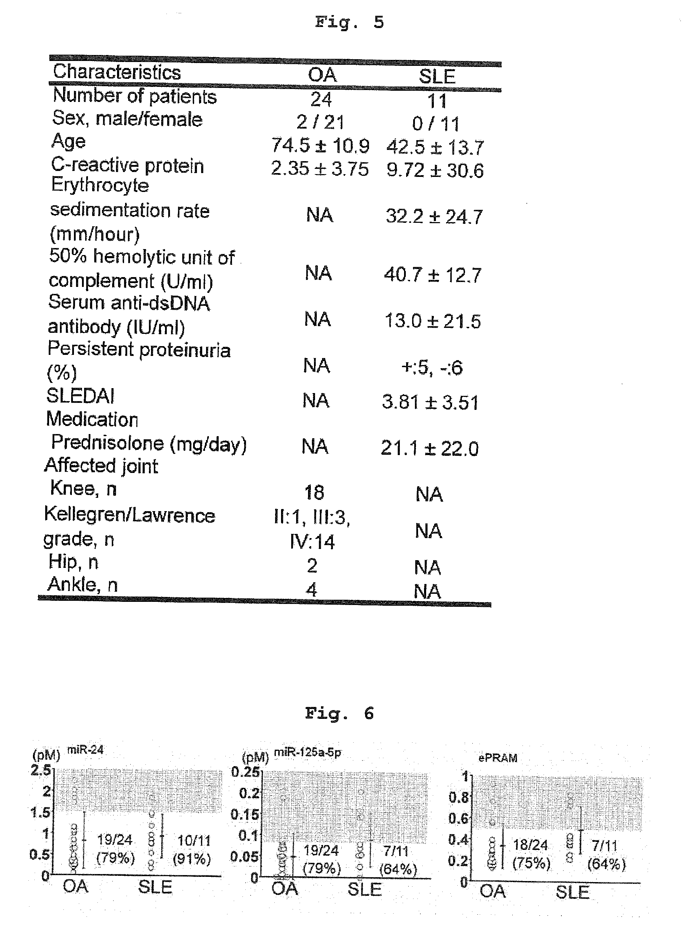

Fig. 5 shows clinical characteristics of osteoarthritis (OA) and systemic lupus erythematosus (SLE) patients.
Fig. 6 shows distribution of concentrations(pM) of miR-24 and miR-125a-5p, and distribution of composite values (ePRAM) of miR-24 and miR-125a-5p in the plasma of osteoarthritis (OA) and systemic lupus erythematosus (SLE) patients. The gray region shows the concentration of each miRNA and ePRAM of the test subjects diagnosed with rheumatoid arthritis.

### Description of Embodiments

### 1. Test method of a sample derived from the blood of a test subject for determining the pathology of rheumatoid arthritis

The present invention provides a method of testing a sample derived from the blood of a test subject for determining the pathology of rheumatoid arthritis, comprising a step of (a) measuring an expression level of at least one miRNA selected from the group consisting of miR-24, miR-26a, miR-28-5p, miR-28-3p, miR-30c, miR-30e-3p, miR-125a-5p, miR-126-3p and miR-502-5p, or expression levels of miR-24 and miR-125a-5p, in a sample derived from the blood of the test subject, and normalizing the expression level(s) with an expression level of internal standard miRNA in the sample (hereinafter sometimes to be also referred to as the test method of the present invention).

In the above-mentioned step (a), preferably, the expression levels of miR-24 and miR-125a-5p alone are measured. More preferably, the expression level is normalized using the expression level of internal standard miRNA. Most preferably, the expression levels of miR-24, miR-30a-5p and miR-125a-5p are measured using miR-30a-5p as the internal standard.

The method of the present invention is generally performed in vitro.

In the present invention, determination of the pathology of rheumatoid arthritis means determination of the stage of progression of the symptoms of rheumatoid arthritis the test subject is in, and includes not only determination of progression of the symptoms of rheumatoid arthritis (i.e., state of the disease) of the test subject, but also determination of the onset of rheumatoid arthritis in the test subject, and determination of the prognosis of rheumatoid arthritis. The test method of the present invention is based on the findings of, in rheumatoid arthritis patients, correlation between the expression levels of miR-24, miR-26a, miR-30c, miR-30e-3p, miR-126-3p and miR-502-5p and conventional indices of rheumatoid arthritis (VAS, DAS28 (ESR) and DAS28 (CRP)), and miRNAs (miR-24, miR-26a, miR-28-5p, miR-28-3p, miR-30c, miR-125a-5p, miR-126-3p and miR-502-5p) having an expression level, which is significantly higher than that of healthy human, in the plasma of rheumatoid arthritis patients, and the pathology of rheumatoid arthritis can be determined with high accuracy by examining the sample derived from the blood of a test subject.

The present applicant has shown that the above-mentioned miRNAs showing a significantly higher expression level than healthy humans in the plasma of rheumatoid arthritis patients are not influenced by destruction of cartilage tissue and general systemic autoimmune reactions, that is, in osteoarthritis and systemic lupus erythematosus, the expression levels of these miRNAs are not significantly different from those of healthy individuals. Accordingly, it is possible to determine that the subject has rheumatoid arthritis but not osteoarthritis or systemic lupus erythematosus by measuring the expression levels of the miRNAs of the present invention.

Therefore, in further aspect, according to the present invention, it is possible to determine the stage of progression of the symptoms of rheumatoid arthritis the test subject is in, and determine that the subject is not affected with osteoarthritis or systemic lupus erythematosus. The miRNAs available as the indices for the determination are miR-24, miR-26a, miR-28-5p, miR-28-3p, miR-30c, miR-30e-3p, miR-125a-5p, miR-126-3p and miR-502-5p, preferably, miR-24 and miR-125a-5p.

The test subject in the present invention may be any mammal. Preferred is a mammal affected with rheumatoid arthritis or suspected to be affected therewith. Examples of the mammal include experiment animals such as rodents such as mouse, rat, hamster, guinea pig and the like, rabbit and the like, companion animals such as dog and cat and the like, domestic animals such as bovine, swine, goat, horse, sheep and the like, primates such as human, monkey, orangutan, chimpanzee and the like, and the like, and human is particularly preferable. The test subject may or may not show the pathology of rheumatoid arthritis, and may or may not be under treatment of rheumatoid arthritis.

In a preferable embodiment of the present invention, the test subject is anti-CCP antibody-negative. As shown in the below-mentioned Examples, the expression levels of miR-24, miR-26a and miR-125a-5p do not differ significantly between anti-CCP antibody-positive case and negative case. Therefore, even when the test subject is anti-CCP antibody-negative, the pathology of rheumatoid arthritis can be determined based on the expression levels of these miRNAs.

Examples of the sample derived from the blood of a test subject include blood, serum, plasma and the like, and serum and plasma are preferable from the aspects of easy preparation of samples, detection sensitivity and the like. Blood-derived samples can be prepared by a method known per se from peripheral blood samples collected from test subjects. When time is necessary from blood sampling to testing, the blood-derived samples (excluding blood) can also be cryopreserved (e.g., -20°C).

In step (a), the expression level of at least one miRNA selected from the group consisting of miR-24, miR-26a, miR-28-5p, miR-28-3p, miR-30c, miR-30e-3p, miR-125a-5p, miR-126-3p and miR-502-5p, or expression levels of miR-24 and miR-125a-5p (preferably, expression level of at least one miRNA selected from the group consisting of miR-24, miR-26a and miR-125a-5p, or expression levels of miR-24, miR-30a-5p and miR-125a-5p, more preferably, expression levels of miR-24, miR-30a-5p and miR-125a-5p), in a sample derived from the blood of a test subject, is measured. In consideration of the fact that miR-30a-5p is an internal standard, moreover, measurement of the expression levels of miR-24 and miR-125a-5p is also preferable, and measurement of the expression levels of miR-24, miR-30a-5p and miR-125a-5p, including miR-30a-5p confirmed to function as the internal standard, is most preferable. These miRNAs are already-known molecules, and a molecule corresponding to each biological species exists. The sequence information is available from databases such as miRBase (http://www.mirbase.org/) and the like. In the present specification, miR-24, miR-26a, miR-28-5p, miR-28-3p, miR-30a-5p, miR-30c, miR-30e-3p, miR-125a-5p, miR-126-3p and miR-502-5p mean molecules naturally present in the biological species of the test subject. For example, when the test subject in the present invention is human, miR-24, miR-26a, miR-28-5p, miR-28-3p, miR-30a-5p, miR-30c, miR-30e-3p, miR-125a-5p, miR-126-3p and miR-502-5p are, as shown in the following Table 1, hsa-miR-24 (consisting of nucleotide sequence shown in SEQ ID NO: 1, registered in miRBase as Accession No. MIMAT0000080 (also called has-miR-24-3p)), hsa-miR-26a (consisting of nucleotide sequence shown in SEQ ID NO: 2, registered in miRBase as Accession No. MIMAT0000082 (also called has-miR-26a-5p)), hsa-miR-28-5p (consisting of nucleotide sequence shown in SEQ ID NO: 3, registered in miRBase as Accession No. MIMAT0000085), hsa-miR-28-3p (consisting of nucleotide sequence shown in SEQ ID NO: 4, registered in miRBase as Accession No. MIMAT0004502), has-miR-30a-5p (consisting of nucleotide sequence shown in SEQ ID NO: 10, registered in miRBase as Accession No. MIMAT0000087), hsa-miR-30c (consisting of nucleotide sequence shown in SEQ ID NO: 5, registered in miRBase as Accession No. MIMAT0000244 (also called has-miR-30c-5p)), hsa-miR-30e-3p (consisting of nucleotide sequence shown in SEQ ID NO: 6, registered in miRBase as Accession No. MIMAT0000693), hsa-miR-125a-5p (consisting of nucleotide sequence shown in SEQ ID NO: 7, registered in miRBase as Accession No. MIMAT0000443), hsa-miR-126-3p (consisting of nucleotide sequence shown in SEQ ID NO: 8, registered in miRBase as Accession No. MIMAT0000445) and hsa-miR-502-5p (consisting of nucleotide sequence shown in SEQ ID NO: 9, registered in miRBase as Accession No. MIMAT0002873), respectively.

**Table 1**

| **sequence name** | **miRBase Accession No.** | **sequence (5'>3')** | **SEQ ID NO** |
|---|---|---|---|
| hsa-miR-24 | MIMAT0000080 | uggcucaguu cagcaggaac ag | 1 |
| hsa-miR-26a | MIMAT0000082 | uucaaguaau ccaggauagg cu | 2 |
| hsa-miR-28-5p | MIMAT0000085 | aaggagcuca cagucuauug ag | 3 |
| hsa-miR-28-3p | MIMAT0004502 | cacuagauug ugagcuccug ga | 4 |
| has-miR-30a-5p | MIMAT0000087 | uguaaacauc cucgacugga ag | 10 |
| hsa-miR-30c | MIMAT0000244 | uguaaacauc cuacacucuc agc | 5 |
| hsa-miR-30e-3p | MIMAT0000693 | cuuucagucg gauguuuaca gc | 6 |
| hsa-miR-125a-5p | MIMAT0000443 | ucccugagac ccuuuaaccu guga | 7 |
| hsa-miR-126-3p | MIMAT0000445 | ucguaccgug aguaauaaug cg | 8 |
| hsa-miR-502-5p | MIMAT0002873 | auccuugcua ucugggugcu a | 9 |

Each miRNA of miR-24, miR-26a, miR-28-5p, miR-28-3p, miR-30a-5p, miR-30c, miR-30e-3p, miR-125a-5p, miR-126-3p and miR-502-5p may contain mutation due to naturally-occurring polymorphism, mutation and the like (substitution, deletion, insertion or addition of nucleotide), and therefore, the expression level of miRNA containing such mutation may be measured in step (a).

The expression level of miRNA can be measured by a method known per se such as hybridization, polymerase chain reaction (PCR) and the like and using a nucleic acid probe or nucleic acid primer capable of specifically detecting each miRNA or a complementary nucleic acid thereof (cRNA or cDNA). Examples of the measurement method include miRNA array, Northern blotting, quantitative PCR (real-time PCR etc.) and the like.

The expression level of miRNA is measured using, as a target, miRNA extracted from a sample derived from the blood of a test subject. The method of extracting miRNA is not particularly limited as long as it is a method of extracting RNA containing miRNA (e.g., total RNA) from a sample derived from the blood of a test subject, and RNA containing miRNA can be extracted using, for example, commercially available reagents and kits (e.g., phenol-based reagent Tripure Isolation Reagent (Roche Applied Science), phenol-based reagent for liquid sample ISOGEN-LS (Nippongene) etc.) according to the protocols attached thereto.

It is also possible to extract RNA containing miRNA from a sample derived from the blood of a test subject and then further isolate miRNA. miRNA can be isolated by a method known per se and using, for example, commercially available reagents and kits (e.g., RNeasy Mini Column (Qiagen), High Pure miRNA Isolation Kit (Roche Applied Science) etc.) according to the protocols attached thereto. Here, "isolation of miRNA" means an operation to remove components other than miRNA.

In the measurement of the expression level of miRNA, to normalize variation between samples due to the above-mentioned extraction and isolation of miRNA, the expression level of miRNA is preferably normalized based on an appropriate internal standard. The internal standard is not particularly limited as long as it is any component naturally contained in a sample derived from the blood of a test subject, and known to show no statistically significant difference between rheumatoid arthritis patients and healthy humans. For example, when the blood-derived sample is plasma, miR-93-3p, miR-223-3p, miR-550, miR-339-3p and miR-30a-5p can be used as the internal standard. In one embodiment, in the calculation of the below-mentioned composite values of the expression levels of miR-24, miR-30a-5p and miR-125a-5p in combination, the measurement error of the expression levels of miR-24 and miR-125a-5p are normalized using miR-30a-5p as the internal standard. Alternatively, miRNA naturally absent in a sample derived from the blood of a test subject may be added as an internal standard to the sample prior to the above-mentioned extraction and isolation of miRNA. For example, when the test subject is human, C. elegans miRNA cel-miR-39 can be used as the internal standard.

In the present specification, that a nucleic acid probe is "capable of specifically detecting miRNA or a complementary nucleic acid thereof" means that the nucleic acid probe hybridizes to the miRNA or a complementary nucleic acid thereof under appropriate hybridization conditions, but does not hybridize to other nucleic acids, since it has higher affinity to the miRNA or a complementary nucleic acid thereof than to a nucleic acid other than the miRNA or a complementary nucleic acid thereof.

Such hybridization conditions can be appropriately selected by those of ordinary skill in the art. As the hybridization conditions, for example, low stringent conditions can be mentioned. The low stringent conditions are, in washing after hybridization, for example, conditions of 42°C, 5×SSC, 0.1% SDS, preferably 50°C, 2×SSC, 0.1% SDS. More preferable hybridization conditions are high stringent conditions. The high stringent conditions are, for example, 65°C, 0.1×SSC, 0.1%SDS. The factors influencing the stringency of hybridization include plural factors of temperature, salt concentration and the like, and those of ordinary skill in the art can realize similar stringency by appropriately selecting these factors.

Examples of the nucleic acid probe capable of specifically detecting a miRNA or a complementary nucleic acid thereof include a polynucleotide containing a continuous nucleotide sequence of not less than about 10 bases (e.g., 10-24 bases, preferably 15 - 24 bases), which is complementary to a part of or whole nucleotide sequence of the miRNA, or a complementary sequence thereof, and capable of hybridizing to the miRNA or a complementary nucleic acid thereof. Such nucleic acid probe can be appropriately designed by a method known per se, based on the information of the nucleotide sequences described in the present specification and the like. For example, miRNA consisting of each nucleotide sequence shown in SEQ ID NOs: 1 - 10 or a complementary nucleic acid thereof can be each detected using, as a nucleic acid probe, a polynucleotide containing each nucleotide sequence shown in SEQ ID NOs: 11 - 20 or a complementary sequence thereof.

In the present specification, a nucleic acid primer being "capable of specifically detecting a miRNA or a complementary nucleic acid thereof" means that the nucleic acid primer amplifies a part of or whole region thereof by PCR under appropriate PCR reaction conditions and using the miRNA or a complementary nucleic acid thereof as a template, but does not amplify a part of or whole region of a nucleic acid other than the miRNA or the complementary nucleic acid thereof by PCR using said nucleic acid as a template.

A nucleic acid primer capable of specifically detecting miRNA may be any as long as it is designed to be able to specifically amplify a part of or whole region of the nucleotide sequence of the miRNA or a complementary nucleic acid thereof. Quantification of miRNA by PCR is generally performed by adding a poly(A) sequence to the 3'terminal by poly(A)polymerase, synthesizing cDNA by reverse transcription reaction, and quantifying the cDNA. In this case, a part of or whole region of the nucleotide sequence of each miRNA or a complementary nucleic acid thereof can be specifically amplified by a combination of a polynucleotide (forward primer) containing a nucleotide sequence of not less than about 10 bases (e.g., 10 - 24 bases, preferably 15 - 24 bases), which hybridizes to a part of the nucleotide sequence of a complementary nucleic acid (cDNA) of each miRNA, and a polynucleotide (reverse primer) containing a nucleotide sequence of not less than about 10 bases (e.g., 10 - 24 bases, preferably 15 - 24 bases), which hybridizes to a part of a complementary sequence of the cDNA sequence on the 3'-side from the hybridization site of the forward primer. The forward primer and reverse primer can be appropriately designed by a method known per se based on information such as the nucleotide sequence of each miRNA or a complementary nucleic acid thereof, the sequence of a primer used for the aforementioned reverse transcription reaction and the like. For specific amplification of a complementary nucleic acid of the miRNA consisting of each nucleotide sequence shown in SEQ ID NO: 1 - 10, for example, a polynucleotide containing each nucleotide sequence shown in SEQ ID NO: 11 - 20 can be used as a forward primer.

The nucleic acid probe and nucleic acid primer may contain an additional sequence (nucleotide sequence not complementary to the polynucleotide to be the detection target) as long as specific detection is not interfered with.

The nucleic acid probe and nucleic acid primer may be labeled with a suitable label, for example, radioisotope (e.g., ¹²⁵I, ¹³¹I, ³H, ¹⁴C, ³²P, ³³P, ³⁵S etc.), enzyme (e.g., β-galactosidase, β-glucosidase, alkaline phosphatase, peroxidase, malic acid dehydrogenase etc.), fluorescent substance (e.g., fluorescamine, fluorescein isothiocyanate etc.), luminescence substance (e.g., luminol, luminol derivative, luciferin, lucigenin etc.), biotin and the like. Alternatively, a quencher (quenching substance) that absorbs fluorescence energy emitted by the fluorescent substance may be further bonded in the vicinity of the fluorescent substance (e.g., FAM, VIC etc.). In such embodiment, the fluorescent substance and the quencher are separated during detection reaction and fluorescence is detected.

The nucleic acid probe and nucleic acid primer may be DNA or RNA, or DNA/RNA chimera, with preference given to DNA. Also, the nucleic acid probe and nucleic acid primer may be single strand or double strand, and generally, single strand.

The nucleic acid probe and nucleic acid primer may be a derivative of nucleic acid having artificial modification. Examples of the nucleic acid derivatives having artificial modification include, but are not limited thereto, those having modified phosphate backbone such as phosphorothioate-type, boranophosphate-type DNA/RNA and the like; 2'-modified nucleotides such as 2'-OMe modified RNA, 2'-F modified RNA and the like; modified nucleotides wherein sugar molecules of nucleotide are crosslinked such as LNA (Locked Nucleic Acid), ENA (2'-O,4'-C-Ethylene-bridged nucleic acids) and the like; modified nucleotides having different basic skeleton such as PNA (peptide nucleic acid), morpholino nucleotide and the like; base-modified nucleotides such as 5-fluorouridine, 5-propyluridine and the like; and the like.

The above-mentioned nucleic acid probe and nucleic acid primer can be synthesized based on, for example, the information of nucleotide sequence described in the present specification and according to a conventional method using a DNA/RNA automatic synthesizer.

A nucleic acid probe capable of specifically detecting the measurement target miRNA or a complementary nucleic acid thereof may be bound on an appropriate support and provided as a nucleic acid array. The support is not particularly limited as long as it is generally used in the pertinent field and, for example, membrane (e.g., nylon membrane), bead, glass, plastic, metal and the like can be mentioned.

The method of the present invention may contain the following step (b):
(b) correlating the expression level measured in step (a) with the pathology of rheumatoid arthritis.

In step (b), the expression level of the miRNA in the sample derived from the blood of the test subject as measured in step (a) is correlated with the pathology of rheumatoid arthritis.

That the expression level of the miRNA in the sample derived from the blood of the test subject is correlated with the pathology of rheumatoid arthritis means determination of whether or not the data from the test subject obtained in step (a) suggest (or indicate) the pathology of rheumatoid arthritis. The data from the test subject is generally correlated with the pathology of rheumatoid arthritis by comparison of the data from the test subject with the data obtained from rheumatoid arthritis patients, comparison of the data from the test subject with the data obtained from non-rheumatoid arthritis patients, or comparison of the data from the test subject with the data obtained from healthy humans. When the data of from test subject does not show a statistically significant difference (generally, p<0.05) from the data from rheumatoid arthritis patients, the data from the test subject can be correlated with rheumatoid arthritis. When the data from the test subject shows a statistically significant difference (generally, p<0.05) from the data from non-rheumatoid arthritis patients or healthy humans, the data from the test subject can be correlated with rheumatoid arthritis. As a result, it is possible to determine that the test subject is affected with rheumatoid arthritis, determine the rate of progression of the symptoms of rheumatoid arthritis of the test subject (i.e., state of the disease), and determine good/poor prognosis of rheumatoid arthritis.

Those of ordinary skill in the art can appropriately correlate the aforementioned expression level of miRNA with the pathology of rheumatoid arthritis without undue trial and error.

As shown in the below-mentioned Examples, the expression levels of miR-24, miR-26a, miR-28-5p, miR-28-3p, miR-30c, miR-125a-5p, miR-126-3p and miR-502-5p in rheumatoid arthritis patients were significantly (p<0.05) higher than healthy human. In addition, the composite value combining the expression levels of miR-24, miR-30a-5p and miR-125a-5p in rheumatoid arthritis patients was significantly (p<0.05) higher than healthy human. That is, when the expression level of at least one miRNA selected from the group consisting of miR-24, miR-26a, miR-28-5p, miR-28-3p, miR-30c, miR-125a-5p, miR-126-3p and miR-502-5p, expression levels of miR-24, miR-30a-5p and miR-125a-5p, or expression levels of miR-24 and miR-125a-5p (preferably, expression level of at least one miRNA selected from the group consisting of miR-24, miR-26a and miR-125a-5p, expression levels of miR-24, miR-30a-5p and miR-125a-5p, or expression levels of miR-24 and miR-125a-5p, more preferably, expression levels of miR-24, miR-30a-5p and miR-125a-5p, or expression levels of miR-24 and miR-125a-5p), in a sample derived from the blood of a test subject, is/are statistically significantly (generally, p<0.05) higher than the data of non-rheumatoid arthritis patients or healthy humans, the test subject can be determined to be affected with rheumatoid arthritis.

To establish the above-mentioned correlation, the expression level of at least one miRNA selected from the group consisting of miR-24, miR-26a, miR-28-5p, miR-28-3p, miR-30c, miR-125a-5p, miR-126-3p and miR-502-5p, expression levels of miR-24, miR-30a-5p and miR-125a-5p, or expression levels of miR-24 and miR-125a-5p (preferably, expression level of at least one miRNA selected from the group consisting of miR-24, miR-26a and miR-125a-5p, expression levels of miR-24, miR-30a-5p and miR-125a-5p, or expression levels of miR-24 and miR-125a-5p, more preferably, expression levels of miR-24, miR-30a-5p and miR-125a-5p, or expression levels of miR-24 and miR-125a-5p), in a measured sample derived from the blood of a test subject, may be compared with the mean value of the previously-determined expression levels of miRNA in many patients who developed rheumatoid arthritis, or many healthy humans.

It is also possible to previously set the cutoff values of the expression level of at least one miRNA selected from the group consisting of miR-24, miR-26a, miR-28-5p, miR-28-3p, miR-30c, miR-125a-5p, miR-126-3p and miR-502-5p, expression levels of miR-24, miR-30a-5p and miR-125a-5p, or expression levels of miR-24 and miR-125a-5p (preferably, expression level of at least one miRNA selected from the group consisting of miR-24, miR-26a and miR-125a-5p, expression levels of miR-24, miR-30a-5p and miR-125a-5p, or expression levels of miR-24 and miR-125a-5p, more preferably, expression levels of miR-24, miR-30a-5p and miR-125a-5p, or expression levels of miR-24 and miR-125a-5p), in a sample derived from blood, and compare the data from the test subject with the cutoff values. For example, when the expression level of miRNA in the sample derived from the blood of a test subject is not less than the cutoff value, the data from the test subject is correlated with the pathology of rheumatoid arthritis, and the test subject can be determined to have rheumatoid arthritis.

The "cutoff value" is a value satisfying both a high diagnostic sensitivity (true positive rate) and a high diagnosis specificity (true negative rate) when a disease is determined using the value as a standard. For example, a value showing a high positive rate in an individual who developed rheumatoid arthritis and a high negative rate in an individual who has not developed rheumatoid arthritis can be determined as a cutoff value.

The calculation method of the cutoff value is well known in the pertinent field. For example, the expression level of at least one miRNA selected from the group consisting of miR-24, miR-26a, miR-28-5p, miR-28-3p, miR-30c, miR-125a-5p, miR-126-3p and miR-502-5p, expression levels of miR-24, miR-30a-5p and miR-125a-5p, or expression levels of miR-24 and miR-125a-5p (preferably, expression level of at least one miRNA selected from the group consisting of miR-24, miR-26a and miR-125a-5p, or expression levels of miR-24, miR-30a-5p and miR-125a-5p, more preferably, expression levels of miR-24, miR-30a-5p and miR-125a-5p, further more preferably, expression levels of miR-24 and miR-125a-5p), in samples derived from the blood of individuals who developed rheumatoid arthritis and individuals who has not developed rheumatoid arthritis are measured, diagnostic sensitivity and diagnosis specificity of the measured values are determined, and an ROC (Receiver Operating Characteristic) curve based on these values is drawn using a commercially available analysis soft. Then, a value at which the diagnostic sensitivity and the diagnosis specificity are as close as possible to 100% is determined, and the value can be used as the cutoff value.

In rheumatoid arthritis patients, moreover, the expression levels of miR-24, miR-26a, miR-30c, miR-30e-3p, miR-126-3p and miR-502-5p tended to become high as the numerical values of the conventional indices of rheumatoid arthritis (CRP, ESR, VAS, DAS28 (ESR) and DAS28 (CRP)) increased. That is, when the test subject is a rheumatoid arthritis patient, the state of the disease can be determined based on the positive correlation between the expression level of at least one miRNA selected from the group consisting of miR-24, miR-26a, miR-30c, miR-30e-3p, miR-126-3p and miR-502-5p in a sample derived from the blood of the test subject and the conventional index of rheumatoid arthritis. For example, when the expression level of at least one miRNA selected from the group consisting of miR-24, miR-26a, miR-30c, miR-30e-3p, miR-126-3p and miR-502-5p in a sample derived from the blood of the test subject is relatively high, the symptoms of rheumatoid arthritis can be determined to have progressed further. In this case, the relative expression level of the miRNA of the test subject is examined utilizing distribution chart of the expression level of the miRNA in samples derived from the blood of many rheumatoid arthritis patients and the like, and the state of the disease can be determined.

In the test method of the present invention, the expression level of at least one miRNA selected from the group consisting of miR-24, miR-26a, miR-28-5p, miR-28-3p, miR-30c, miR-30e-3p, miR-125a-5p, miR-126-3p and miR-502-5p (preferably, at least one miRNA selected from the group consisting of miR-24, miR-26a and miR-125a-5p, more preferably, at least one miRNA selected from the group consisting of miR-24 and miR-125a-5p), in a sample derived from the blood of a test subject, only needs to be measured. It is sometimes possible to highly accurately determine the pathology of rheumatoid arthritis by measuring the expression levels of plural (e.g., two or more, preferably not less than 3) miRNAs therefrom, and correlating same with the pathology of rheumatoid arthritis. As shown in the below-mentioned Examples, moreover, it is possible to particularly highly accurately determine the presence or absence of affection with rheumatoid arthritis based on the expression levels of miR-24 and miR-125a-5p from these miRNAs and the expression level of internal standard miR-30a-5p. When the expression levels of plural miRNAs are measured and correlated with the pathology of rheumatoid arthritis, they can be correlated using a composite value of the plural miRNAs. For example, when the "composite value (ePRAM) of miR-24, miR-30a-5p and miR-125a-5p" is to be determined, it can be calculated from ePRAM=exp(-x)/(1+exp(-x)), namely, x=1.797 - 0.773×miR-24 + 0.141xmiR-30a-5p - 15.6×miR-125a-5p, based on the expression level (unit pM) of each miRNA.

The composite value (ePRAM) can be analyzed by any method as long as the desired effect can be obtained. For example, the calculation formula obtained by the multivariate logistic regression analysis can be used for the analysis. miRNAs that can be the target when the disease state of rheumatoid arthritis is subjected to the multivariate logistic regression analysis includes miR-24, miR-26a, miR-28-5p, miR-28-3p, miR-30c, miR-125a-5p, miR-126-3p and miR-30a-5p, and the expression levels of these can be combined for analysis. miRNAs which can be the target are preferably miR-24, miR-30a-5p and miR-125a-5p.

The composite value may be adjusted by the above-mentioned calculation based on the measurement method, race and age, or miRNA other than those recited above as examples can also be used. As the internal standard, miR-30a-5p or other internal standard miRNA may be used, or an internal standard may not be necessary in some cases.

In a preferable embodiment of the test method of the present invention, the above-mentioned miRNA expression level can be combined with an anti-CCP antibody level, which is one of the known test standards for the diagnoses of rheumatoid arthritis to calculate a composite value, based on which the pathology of rheumatoid arthritis can be determined. As shown in the below-mentioned Examples, since the expression levels of miR-24, miR-26a and miR-125a-5p do not differ significantly between anti-CCP antibody-positive cases and negative cases, rheumatoid arthritis in the anti-CCP antibody-negative cases can also be determined based on the expression levels of these miRNAs. Therefore, high sensitivity and specificity can be obtained by combining the expression level of at least one miRNA selected from the group consisting of miR-24, miR-26a and miR-125a-5p in a sample derived from the blood of a test subject and serum anti-CCP antibody level, and correlating the levels with the pathology of rheumatoid arthritis. In addition, as shown in the below-mentioned Examples, particularly high sensitivity and specificity can be obtained by combining the expression levels of miR-24, miR-30a-5p and miR-125a-5p and serum anti-CCP antibody level, and correlating the levels with the pathology of rheumatoid arthritis. Since miR-30a-5p is an internal standard, it is also possible to normalize the expression levels of miR-24 and miR-125a-5p with any internal standard, combine same with serum anti-CCP antibody levels, and correlate the values with the pathology of rheumatoid arthritis.

The anti-CCP antibody level in the serum of a test subject can be measured by a method known per se. For example, it can be measured by ELISA utilizing cyclic citrullinated peptide (CCP) (cyclic peptide containing citrullinated site of filaggrin), which is an antigen to an anti-CCP antibody, (Arthritis Rheum., 43(1): p.155-63(2000), Ann. Rheum. Dis., 64: p.1510-1512(2005)) and the like. Also, a commercially available anti-CCP antibody measurement kit such as MESACUP-2 or MEBChrom CCP test of MBL and the like may be used.

Correlation with the pathology of rheumatoid arthritis can be performed in the same manner as above based on, for example, a composite value (ePRAM-C) ePRAM-C=exp(-x)/(1+exp(-x)) (x=3.075 - 614.386×miR-24 + 80.96×miR-30a-5p - 9292.79×miR-125a-p - 5.856×anti-CCP antibody (positive; 1, negative; 0) can be calculated from the above-mentioned expression levels of miR-24, miR-30a-5p and miR-125a-5p, as well as anti-CCP antibody levels.

As a result of the above-mentioned correlation, when the test subject is determined to be affected with rheumatoid arthritis, an appropriate treatment such as administration of a therapeutic drug for rheumatoid arthritis and the like can be performed. When the disease state of rheumatoid arthritis of the test subject is determined to have progressed, an appropriate treatment such as an increase in the dose of a therapeutic drug for rheumatoid arthritis and the like can be performed. On the other hand, when the disease state of rheumatoid arthritis of the test subject is determined to have weakened, an appropriate treatment such as a decrease in the dose of a therapeutic drug for rheumatoid arthritis and the like can be performed.

In a further aspect, when the test subject is determined to have been affected with rheumatoid arthritis based on the above-mentioned correlation, the test subject can be simultaneously determined to have been not affected with osteoarthritis or systemic lupus erythematosus. For such determination, the expression level of at least one miRNA selected from the group consisting of miR-24, miR-26a, miR-28-5p, miR-28-3p, miR-30c, miR-30e-3p, miR-125a-5p, miR-126-3p and miR-502-5p, or expression levels of miR-24 and miR-125a-5p, preferably miRNA expression level(s) of miR-24 and/or miR-125a-5p are/is measured and can be correlated by the above-mentioned method.

### 2. Diagnostic reagent for determining the pathology of rheumatoid arthritis

The present invention provides a diagnostic reagent for determining the pathology of rheumatoid arthritis, comprising a nucleic acid probe or nucleic acid primer capable of specifically detecting at least one miRNA selected from the group consisting of miR-24, miR-26a, miR-28-5p, miR-28-3p, miR-30c, miR-30e-3p, miR-125a-5p, miR-126-3p and miR-502-5p, or a nucleic acid probe or nucleic acid primer capable of specifically detecting miR-24 and a nucleic acid probe or nucleic acid primer capable of specifically detecting miR-125a-5p. In a preferable embodiment, the present invention provides a diagnostic reagent for determining the pathology of rheumatoid arthritis, comprising a nucleic acid probe or nucleic acid primer capable of specifically detecting miR-24, and a nucleic acid probe or nucleic acid primer capable of specifically detecting miR-125a-5p, wherein the diagnostic reagent may contain any nucleic acid probe or nucleic acid primer capable of specifically detecting internal standard miRNA. As a preferable internal standard miRNA, miR-30a-5p can be mentioned. Using the diagnostic reagent of the present invention, the above-mentioned test method of the present invention can be performed easily, and the pathology of rheumatoid arthritis can be determined rapidly with high accuracy.

The detail of the nucleic acid probe or nucleic acid primer to be contained in the diagnostic reagent of the present invention is as described in "1. Test method of sample derived from the blood of a test subject for determining the pathology of rheumatoid arthritis". To be more specific, the diagnostic reagent of the present invention contains a nucleic acid probe or nucleic acid primer capable of specifically detecting at least one miRNA selected from the group consisting of miR-24, miR-26a, miR-28-5p, miR-28-3p, miR-30c, miR-30e-3p, miR-125a-5p, miR-126-3p and miR-502-5p; a nucleic acid probe or nucleic acid primer capable of specifically detecting miR-24, a nucleic acid probe or nucleic acid primer capable of specifically detecting miR-30a-5p, and a nucleic acid probe or nucleic acid primer capable of specifically detecting miR-125a-5p; or a nucleic acid probe or nucleic acid primer capable of specifically detecting miR-24, and a nucleic acid probe or nucleic acid primer capable of specifically detecting miR-125a-5p, preferably, a nucleic acid probe or nucleic acid primer capable of specifically detecting at least one miRNA selected from the group consisting of miR-24, miR-26a and miR-125a-5p; a nucleic acid probe or nucleic acid primer capable of specifically detecting miR-24, a nucleic acid probe or nucleic acid primer capable of specifically detecting miR-30a-5p, and a nucleic acid probe or nucleic acid primer capable of specifically detecting miR-125a-5p; or a nucleic acid probe or nucleic acid primer capable of specifically detecting miR-24, and a nucleic acid probe or nucleic acid primer capable of specifically detecting miR-125a-5p, more preferably, a nucleic acid probe or nucleic acid primer capable of specifically detecting miR-24, a nucleic acid probe or nucleic acid primer capable of specifically detecting miR-30a-5p, and a nucleic acid probe or nucleic acid primer capable of specifically detecting miR-125a-5p; or a nucleic acid probe or nucleic acid primer capable of specifically detecting miR-24, and a nucleic acid probe or nucleic acid primer capable of specifically detecting miR-125a-5p.

The diagnostic reagent of the present invention may be provided as a nucleic acid array by binding a nucleic acid probe capable of specifically detecting each measurement target miRNA or a complementary nucleic acid thereof onto an appropriate support. The support is not particularly limited as long as it is generally used in the pertinent field, and examples thereof include membrane (e.g., nylon membrane), bead, glass, plastic, metal and the like.

Each constituent element contained in the diagnostic reagent of the present invention is dissolved separately (or in a mixed state when possible) in water or a suitable buffer (e.g., TE buffer, PBS etc.) to a suitable concentration, or placed in an appropriate container in a freeze-dried state.

According to the measurement method of the miRNA expression level, the diagnostic reagent of the present invention may further contain, in its constitution, other components necessary for performing the method. The pathology of rheumatoid arthritis can be determined by measuring the miRNA expression level by, for example, miRNA array, Northern blotting, quantitative PCR (real-time PCR etc.) and the like, by the diagnosis drub of the present invention. When real-time PCR is used for the measurement, the diagnostic reagent of the present invention can further contain 10×PCR reaction buffer, 10×MgCl₂ aqueous solution, 10×dNTPs aqueous solution, Taq DNA polymerase (5 U/µL), reverse transcriptase and the like. When miRNA array and Northern blotting are used for the measurement, the diagnostic reagent of the present invention can further contain blotting buffer, labeling reagent, blotting membrane and the like.

In addition, the diagnostic reagent of the present invention can further contain, in its constitution, other components necessary for the measurement of the anti-CCP antibody level. Such other component is known in the pertinent field. For example, when the anti-CCP antibody level is measured by an immunological method such as ELISA and the like, the diagnostic reagent of the present invention can contain cyclic citrullinated peptide. The cyclic citrullinated peptide may be immobilized on a carrier (e.g., membrane, bead, glass, plastic, metal etc.). In addition to the cyclic citrullinated peptide, a labeled anti-IgG antibody capable of binding to the anti-CCP antibody of the test subject, and the like may be further contained, whereby a preferable embodiment of the test method of the present invention including a step of measuring the serum anti-CCP antibody levels of the test subject can be efficiently performed.

### Examples

The Examples of the present invention are explained in the following; however, the present invention is not imitated by these Examples in any way.

### Method

### (1) Preparation of plasma sample

This research was approved by the ethics committee of Graduate School of Medicine and Faculty of Medicine, Kyoto University. Informed consent was obtained from 206 participants (102 rheumatoid arthritis patients (RA), 104 healthy individuals (HC)). RA was diagnosed according to the standard of American College of Rheumatology (Arthritis Rheum 1988, 31:315-324). Peripheral blood was collected from 102 RAs. The blood samples were placed in a tube containing EDTA-2K and plasma was separated. The samples were centrifuged for 7 min at 1400 g and cryopreserved at -80°C before analysis.

### (2) Preparation of total RNA

The plasma (4 ml) was thawed on ice, diluted with RNase Free Water (4 ml), and extracted twice with phenol-based reagent Sepasol-RNA II (Nacalai Tesque) for liquid sample. To normalize dispersion between samples caused by RNA isolation, synthesized C. elegans miRNA cel-miR-39 (1 pmol, 20 µl, Hokkaido System Science) (homologous sequence nonexistent in human) was added to each denatured sample. The obtained liquid samples were applied to RT2-qPCR-Grade miRNA isolation kit (SABiosciences), and RNA was extracted according to the protocol of the manufacturer.

### (3) Isolation of miRNA

The plasma (150 µl) was thawed on ice, and dissolved in 600 µl of phenol-based reagent Tripure Isolation Reagent (Roche Applied Science). To normalize dispersion between samples caused by RNA isolation, synthesized C. elegans miRNA cel-miR-39 (25 fmol, 5 µl, Hokkaido System Science) (homologous sequence is not present in human) was added to each denatured sample. The samples were stood for 5 min, chloroform (0.15 ml) was added, and the mixture was vigorously shaken for 15 sec. After standing for 3 min, the mixture was centrifuged at 4°C for 15 min at 12,000 g. The aqueous phase (400 µl) was separated to prepare total RNA. Then, the aqueous phase (400 µl) was mixed with ethanol (600 µl), and RNA was bound to RNeasy Mini Column (Qiagen). The column was washed once with Buffer RWT (700 µl, Qiagen) and three times with Buffer RPE (500 µl, Qiagen), and miRNA was isolated with RNase Free Water (37.5 µl).

### (4) miRNA array analysis

Using TaqMan human miRNA array pool A v3.0, B v2.0 (Life Technologies) and Applied BioSystems 7900 Real-Time PCR system (Life Technologies), the total RNA was subjected to miRNA array analysis.

### (5) Reverse transcription of miRNA and quantitatively real-time PCR

Reverse transcription was performed by using NCode VILO miRNA cDNA Synthesis Kit (Invitrogen) according to the protocol of the manufacturer. Real-time PCR was performed by using EXPRESS SYBR GreenER qPCR SuperMix Universal (Invitrogen) and Applied BioSystems 7500 Real-Time PCR System (Life Technologies). Forward primer was designed according to the NCode miRNA database (Invitrogen). The primer attached to the aforementioned Kit was used as a reverse primer. The data was analyzed using SDS Relative Quantification Software version 2.06 (Applied BioSystems).

The sequences of the forward primers used are as follows:
for miR-24 amplification, 5'-TGGCTCAGTTCAGCAGGAACA-3' (SEQ ID NO: 11)
for miR-26a amplification, 5'-GATTTCAAGTAATCCAGGATAGGCT-3' (SEQ ID NO: 12)
for miR-28-5p amplification, 5'-GGCAAGGAGCTCACAGTCTATTGAG-3' (SEQ ID NO: 13)
for miR-28-3p amplification, 5'-CACTAGATTGTGAGCTCCTGGA-3' (SEQ ID NO: 14)
for miR-30a-5p amplification, 5'-GCGTGTAAACATCCTCGACTGG-3' (SEQ ID NO: 20)
for miR-30c amplification, 5'-CGGTGTAAACATCCTACACTCTCAGC-3' (SEQ ID NO: 15)
for miR-30e-3p amplification, 5'-CTTTCAGTCGGATGTTTACAGC-3' (SEQ ID NO: 16)
for miR-125a-5p amplification, 5'-TCCCTGAGACCCTTTAACCTGTGA-3' (SEQ ID NO: 17)
for miR-126-3p amplification, 5'-CGCTCGTACCGTGAGTAATAATGCG-3' (SEQ ID NO: 18)
for miR-502-5p amplification, 5'-GCTTATCCTTGCTATCTGGGTGCTA-3' (SEQ ID NO: 19).

miRNA available as the internal standard was searched for, and a plurality of miRNAs showing expression levels close to mean Ct-value of many miRNAs were identified by real-time PCR. Also in an internal standard candidate analysis using geNorm or NormFinder software, a plurality of miRNAs showing expression levels close to the mean value were identified. As the candidate internal standard miRNAs, miR-93-3p, miR-223-3p, miR-339-3p, miR-30a-5p, miR-301a-5p and miR-484-5p were identified. From these candidate internal standard miRNAs, the expression level of miR-30a-5p was used as the internal standard in the Examples.

### (6) Clinical index

VAS (visual analogue scale of general health) is self-evaluation of the general condition of patients. The numerical values scored by the patients on a 100 mm scale, wherein 0="feel very well (no symptom)" and 100="feel very sick", were used. DAS28 (ESR) and DAS28 (CRP) were calculated by a conventional method (e.g., from calculation formula described in http://www.das-score.nl/ and the like).

### (7) Statistical analysis

The expression level of miRNA was shown in mean±standard deviation. The statistical analysis was performed using JMP8 (SAS Japan). The difference between two groups was analyzed by Student's t-test. The correlation between the miRNA concentration and other clinical indices was analyzed by Pearson product-moment correlation coefficient. p<0.05 was considered statistically significant.

### Results

Total RNA was extracted from the plasma of three samples each from the rheumatoid arthritis patients (RA) and healthy humans (HC), and miRNA array was performed. As for 26 miRNAs suggested to have a difference in the expression levels between RA and HC, the difference in the expression levels between 8 samples each of RA and HC was confirmed by quantitative PCR, and 10 miRNAs shown in Table 2 were selected as candidate diagnosis markers or disease state markers. Furthermore, the expression levels of these miRNAs were measured in 102 rheumatoid arthritis patients and 104 healthy individuals, and statistically analyzed.

As a result, as shown in Table 2, miR-24, miR-26a, miR-28-5p, miR-28-3p, miR-30c, miR-125a-5p, miR-126-3p and miR-502-5p in the plasma showed a significant increase in RA as compared to HC.

**Table 2**

| miRNA | (pM) | P value |
|---|---|---|
| miR-24 | | |
| RA | 3.3 ± 3.1 | |
| HC | 0.88 ± 0.79 | 7.2 × 10⁻³ |
| miR-26a | | |
| RA | 5.7 ± 6.4 | |
| HC | 2.0 ± 9.1 | 8.0 × 10⁻⁴ |
| miR-28-5p | | |
| RA | 4.9 × 10⁻² ± 9.7 × 10⁻² | |
| HC | 1.8 × 10⁻² ± 2.6 × 10⁻² | 1.6 × 10⁻³ |
| miR-28-3p | | |
| RA | 7.2 ± 7.3 | |
| HC | 3.3 ± 4.2 | 6.6 × 10⁻⁶ |
| miR-30a-5p | | |
| RA | 4.5 ± 3.5 | |
| HC | 7.7 ± 50 | 0.52 |
| miR-30c | | |
| RA | 2.3 ± 2.0 | |
| HC | 0.91 ± 0.66 | 1.1 × 10⁻¹⁰ |
| miR-30e-3p | | |
| RA | 25 ± 49 | |
| HC | 35 ± 35 | 0.49 |
| miR-125a-5p | | |
| RA | 0.20 ± 0.29 | |
| HC | 0.074 ± 0.41 | 0.01 |
| miR-126-3p | | |
| RA | 13 ± 14 | |
| HC | 3.2 ± 2.8 | 1.0 × 10⁻¹⁰ |
| miR-502-5p | | |
| RA | 1.2 ± 0.41 | |
| HC | 1.4 ± 0.79 | 3.4 × 10⁻⁶ |

The prediction values for RA prediction were calculated by multivariate logistic regression analysis. The expression levels of miR-24, miR-26a, miR-28-5p, miR-28-3p, miR-30c, miR-125a-5p, miR-126-3p and miR-30a-5p were combined, and the values predicting the disease state of rheumatoid arthritis most accurately were analyzed by multivariate logistic regression analysis to find that the calculation by a combination of miR-24, miR-30a-5p and miR-125a-5p predicted most accurately.

miR-24, miR-26a and miR-125a-5p were analyzed by ROC curve analysis. As a result, the Area Under Curve was 0.80, 0.80 and 0.83, respectively, and the sensitivity and specificity in the determination of the presence or absence of affection with rheumatoid arthritis were 63.7%, 89.5% (cutoff value: 1.46 pM); 53.9%, 94.3% (cutoff value: 3.09 pM); and 64.7%, 89.5% (cutoff value: 8.17 x 10⁻² pM) (Fig. 1), respectively.

In addition, based on the composite value ePRAM (based on the expression level of each miRNA (unit: pM), ePRAM=exp(-x)/(1+exp(-x)) wherein x=1.797 - 0.773×miR-24 + 0.141×miR-30a-5p - 15.6×miR-125a-5p,) obtained by combining the expression levels of miR-24, miR-30a-5p and miR-125a-5p, a test with sensitivity of 78.4% and specificity of 92.3% could be performed (Fig. 2).

Furthermore, based on the composite value (ePRAM-C) (ePRAM-C=exp(-x)/(1+exp(-x)) wherein x=3.075 - 614.386×miR-24 + 80.96×miR-30a-5p - 9292.79×miR-125a-p - 5.856×anti-CCP antibody (positive; 1, negative; 0)) obtained by combining the existing anti-CCP antibody level and the expression levels of miR-24, miR-30a-5p and miR-125a-5p, a test with sensitivity of 94.1% and specificity of 95.2% could be performed (Fig. 3).

The expression levels of miR-24, miR-26a and miR-125a-5p showed no significant difference between anti-CCP antibody-positive cases and negative cases (Fig. 4).

The above-mentioned results show that miR-24, miR-26a and miR-125a-5p in plasma can be markers for distinguishing rheumatoid arthritis patients from healthy humans, and that the composite value obtained by combining the expression levels of miR-24, miR-30a-5p and miR-125a-5p, and the composite value obtained by combining the expression levels of miR-24, miR-30a-5p and miR-125a-5p and existing anti-CCP antibody level permit realization of a test with high sensitivity and high specificity.

Furthermore, the rheumatoid arthritis patients were examined for the numerical values of rheumatoid arthritis indices (VAS, DAS28 (ESR) and DAS28 (CRP)), and the correlation with the expression level of miRNA in plasma was statistically tested.

As a result, a correlation between the absolute value of miRNA concentration or logarithm thereof and these rheumatoid arthritis indices was found in miR-24, miR-26a, miR-30c, miR-126-3p and miR-502-5p and VAS; miR-24, miR-26a, miR-30c, miR-30e-3p, miR-126-3p and miR-502-5p and DAS28 (ESR); as well as miR-24, miR-26a, miR-30c, miR-30e-3p, miR-126-3p and miR-502-5p and DAS28 (CRP). ESR and CRP each reflect an inflammation reaction. The miRNA expression level showing a correlation therewith can be one index for examining the profile of an inflammation reaction. In addition, VAS is subjective disease evaluation made by patients, and being correlated therewith means that it can be an index capable of objectively evaluating the subjective evaluation made by patients. Furthermore, DAS28 (ESR) and DAS28 (CRP) reflect disease activity of rheumatoid arthritis. Using these indices, an increase or decrease, change of medicaments and the like are determined at present, and the evaluation of disease activity by the markers correlated therewith can also be utilized for the selection of treatments.

From the above-mentioned results, it has been clarified that the expression levels of miR-24, miR-26a, miR-28-5p, miR-28-3p, miR-30c, miR-30e-3p, miR-125a-5p, miR-126-3p and miR-502-5p in plasma can be used as the disease state markers of rheumatoid arthritis, and can be used for the time-course judgment of disease activity, determination of treatment effects and the like.

### (Comparative Example)

Whether the miRNAs of the present invention, which were found as a disease state marker of rheumatoid arthritis, also shows an increased expression level in osteoarthritis which is a non-inflammatory arthropathy, and systemic lupus erythematosus which is a non-rheumatic autoimmune disease was examined. The information of the test subjects as the investigation target is shown in Fig. 5. The expression levels of miR-24 and miR-125a-5p in plasma, and a composite value of these (ePRAM) were analyzed. Among the osteoarthritis patients, the proportion of the patients who tested negative in the test of miR-24 and miR-125a-5p, and the composite value of these (ePRAM) was 79%, 79% and 75%, respectively (Fig. 6). Among the systemic lupus erythematosus patients, the proportion of the patients who tested negative in the test of miR-24 and miR-125a-5p, and the composite value of these (ePRAM) was 91%, 64% and 64%, respectively (Fig. 6). Therefore, these, results indicate that miR-24 and miR-125a-5p, and the composite value of these (ePRAM) do not show high values in osteoarthritis and systemic lupus erythematosus, that is, they function as markers specific to rheumatoid arthritis.

### Industrial Applicability

According to the present invention, a means for diagnosis of rheumatoid arthritis, which is based on the expression level of miRNA in plasma, is provided. Since the present invention provides a diagnostic reagent of rheumatoid arthritis which can also diagnose anti-CCP antibody negative cases, an early treatment becomes available for more patients. According to the present invention, moreover, patients can be stratified for appropriate selection of the treatment target patients, and the like. Therefore, the use efficiency of medicaments can be enhanced, thus contributing to the medical economy.

This application is based on a patent application No. 2012-186937 filed in Japan (filing date: August 27, 2012), the contents of which are incorporated in full herein.

## Claims

1. A method of testing a sample derived from the blood of a test subject for determining the pathology of rheumatoid arthritis, comprising a step of
(a) measuring an expression level of at least one miRNA selected from the group consisting of miR-24, miR-26a, miR-28-5p, miR-28-3p, miR-30c, miR-30e-3p, miR-125a-5p, miR-126-3p and miR-502-5p, or expression levels of miR-24 and miR-125a-5p, in the sample derived from the blood of the test subject, and normalizing the expression level(s) with an expression level of an internal standard miRNA in the sample.

2. The method according to claim 1, further comprising the following step:
(b) correlating the expression level measured in step (a) with the pathology of rheumatoid arthritis.

3. The method according to claim 1 or 2, wherein, in step (a), an expression level of at least one miRNA selected from the group consisting of miR-24, miR-26a and miR-125a-5p, or expression levels of miR-24 and miR-125a-5p, in the sample derived from the blood of the test subject, is/are measured.

4. The method according to claim 3, wherein, in step (a), expression levels of miR-24 and miR-125a-5p in the sample derived from the blood of the test subject are measured.

5. The method according to claim 3 or 4, further comprising a step of
(a') measuring a level of an anti-CCP antibody in the sample derived from the blood of the test subject,
wherein, in step (b), the expression level measured in step (a) and the anti-CCP antibody level measured in step (a') are correlated with the pathology of rheumatoid arthritis.

6. The method according to claim 3 or 4, wherein the test subject is anti-CCP antibody negative.

7. The method according to any one of claims 1 to 6, wherein the sample derived from the blood is plasma.

8. The method according to any one of claims 1 to 7, wherein the test subject is a human.

9. A diagnostic reagent for determining the pathology of rheumatoid arthritis, comprising a nucleic acid probe or nucleic acid primer capable of specifically detecting at least one miRNA selected from the group consisting of miR-24, miR-26a, miR-28-5p, miR-28-3p, miR-30c, miR-30e-3p, miR-125a-5p, miR-126-3p and miR-502-5p, or
a nucleic acid probe or nucleic acid primer capable of specifically detecting miR-24,
and
a nucleic acid probe or nucleic acid primer capable of specifically detecting miR-125a-5p.

10. The diagnostic reagent according to claim 9, comprising the nucleic acid probe or nucleic acid primer capable of specifically detecting at least one miRNA selected from the group consisting of miR-24, miR-26a and miR-125a-5p, or
the nucleic acid probe or nucleic acid primer capable of specifically detecting miR-24,
and
the nucleic acid probe or nucleic acid primer capable of specifically detecting miR-125a-5p.

11. The diagnostic reagent according to claim 10, comprising the nucleic acid probe or nucleic acid primer capable of specifically detecting miR-24,
and
the nucleic acid probe or nucleic acid primer capable of specifically detecting miR-125a-5p.

12. The diagnostic reagent according to any one of claims 9 to 11, further comprising a cyclic citrullinated peptide.

13. The method according to any one of claims 1 to 8, wherein the internal standard miRNA is miR-30a-5p.

14. The diagnostic reagent according to any one of claims 9 to 12, further comprising a nucleic acid probe or nucleic acid primer capable of specifically detecting miR-30a-5p.
